# EUROPEAN PATENT APPLICATION

(11) **EP 4 801 047 A2**
(43) Date of publication of application: **02.09.2026**
(21) Application number: 26160512.5
(22) Date of filing: 24.02.2026
(51) Int. Cl.: H04N 23/50, A61B 1/00, H04N 23/57

(54) **SIGNAL ADAPTER DEVICE**

(30) Priority: 28.02.2025 US 202563764625 P
(71) Applicant: Karl Storz SE & Co. KG, 78532 Tuttlingen (DE)
(72) Inventor: Wochner, Tobias, 78532 Tuttlingen (DE); Radosevic, Marko, Goleta, 93117 (US); VanRossen, Chase, Goleta, 93117 (US)

(57) **Abstract**

A signal adapter device for interfacing a medical camera with a processing device is disclosed. The signal adapter device comprises a first interface configured to receive video signals from a medical camera and a second interface configured to transmit converted signals to a processing device. A processing unit converts video and control signals between different formats. The signal adapter device includes a mechanical locking mechanism at the second interface to prevent unintentional disconnection, while allowing the first interface to remain freely detachable. The housing incorporates thermal management structures to dissipate heat from internal components and, optionally, from the connected camera. The signal adapter device may further regulate power supplied to the camera, with optional electrical isolation.

## Description

### CROSS-REFERENCE TO RELATED APPLICATIONS

The present application refers to a signal adapter device for interfacing a medical camera with a processing device.

### BACKGROUND

Medical imaging devices, such as endoscopic and surgical cameras, are commonly used in clinical environments to provide real-time visual feedback during medical procedures. These cameras are often designed to output video signals in specific proprietary or legacy formats.

One well-known manufacturer of such medical cameras is Karl Storz SE & Co. KG ("Karl Storz"), which offers a range of medical imaging systems, including models such as the IMAGE1 S HX and the FLEX-XC. These and similar cameras may output video signals in Digital Visual Interface (DVI) or Low-Voltage Differential Signaling (LVDS) formats, or in variations thereof.

Medical cameras of this type are typically connected to a processing device, which receives the video signals for further processing, displaying, or storing. In addition to handling the video signal, such processing devices may also provide power supply to the connected camera and/or transmit control signals to adjust camera settings, such as exposure, focus, or white balance.

As with medical cameras, various manufacturers, including Karl Storz, provide processing devices designed to interface with specific camera models. Such image processing systems include models such as the IMAGE1 S^{™} series (e.g., TC300: H3-Link, TC301: X-Link, TC302: D3-Link, TC304: 4U-Link), and the TELECAM C3 (TC100). These devices ensure seamless integration into clinical workflows and compatibility with corresponding medical cameras.

However, due to advancements in medical imaging technology, newer processing devices may no longer include all of the necessary interfaces to support former medical cameras. As a result, medical professionals may face challenges when attempting to connect legacy cameras to modern imaging systems, potentially limiting the usability of existing medical equipment.

Users seek to continue using their existing medical cameras, endoscopes, and related imaging devices while also benefiting from the improved performance, advanced processing capabilities, and enhanced image quality provided by modern processing devices. This creates a need for a solution that ensures compatibility between former medical cameras and newer processing devices, allowing a seamless transition while maintaining full functionality.

Modern medical imaging systems must balance compatibility between legacy and advanced technologies. As medical professionals seek to integrate newer image processing devices into their workflows, they often encounter difficulties when attempting to connect older medical cameras that use outdated signal formats or proprietary connectors. These challenges arise due to differences in signal protocols, power delivery methods, and control signal compatibility between older and newer devices.

Additionally, mechanical stability is a key consideration in medical environments where accidental disconnection of cables can disrupt procedures. Existing adapters may lack secure locking mechanisms, increasing the risk of unintentional detachment from the processing unit.

Thermal management presents another concern, as signal adapters often house processing units that generate heat during signal conversion. In some cases, medical cameras themselves transfer thermal energy through their connection cables, further increasing the need for efficient heat dissipation within the signal adapter device.

### SUMMARY

To address the needs explained above, the present invention provides a signal adapter device that facilitates compatibility between legacy medical cameras and modern processing devices. The signal adapter device converts video signals between different formats while ensuring a mechanically secure connection and efficient thermal management.

The present disclosure relates to a signal adapter device for medical imaging applications. More specifically, the disclosure pertains to an adapter that enables the conversion of video signals from a medical camera, such as an endoscopic or surgical camera, to a format compatible with an image processing device. The signal adapter device may further incorporate mechanical locking features and thermal management structures to ensure secure and stable operation in clinical environments.

The present disclosure provides a signal adapter device for interfacing a medical camera with a processing device. The signal adapter device enables the conversion of video signals between different formats, allowing medical professionals to continue using legacy imaging devices with modern processing equipment. In addition to signal conversion, the invention incorporates further functional aspects to ensure reliable and stable operation in medical environments.

The following sections describe aspects of the invention. Each aspect may correspond to a particular technical focus, including signal conversion and interface compatibility, mechanical securing and connector design, and thermal management for stable operation.

Each of these aspects may be implemented individually or in combination, depending on the specific requirements of the application.

According to a first aspect, the present invention provides a signal adapter device for interfacing a medical camera with a processing device. The signal adapter device comprises:
a first interface, which is configured to connect to a medical camera via a first connector and to receive video signals in at least one first signaling format;
a second interface, which is configured to connect to a processing device via a second connector and to output converted video signals in a second signaling format, wherein the first signaling format is different from the second signaling format;
a processing unit, which is electrically connected to both interfaces and configured to:
   receive video signals from the medical camera via the first interface;
   transmit converted video signals to the processing device via the second interface;
   process and convert control signals bidirectionally between the first and second interfaces; and
   a housing, which encloses the processing unit and at least a portion of the first and second interfaces.

In some implementations, the first signaling format comprises at least one of Digital Visual Interface (DVI) or Low-Voltage Differential Signaling (LVDS). In some implementations, the second signaling format is based on a Multi-Gigabit Transceiver (MGT) standard.

The processing unit may comprise a field-programmable gate array (FPGA) or another programmable signal converter (e.g., implemented by a microcontroller), which is configured to perform the signal conversion between the first interface and the second interface. In certain implementations, the FPGA or programmable converter is further configured to receive firmware or programming updates via a dedicated programming interface or through the second interface.

The processing unit may also be configured to detect the first signaling format received via the first interface and to adjust the conversion settings accordingly.

In some implementations, the first interface is configured to provide power to the medical camera via the first connector. For this purpose, the signal adapter device may include a voltage converter, which is configured to adjust the voltage received from the second interface to a level suitable for operating the medical camera.

To provide electrical isolation, the signal adapter device may further comprise an isolation circuit, which is arranged between the first and second interfaces.

According to a further aspect, the present invention provides a signal adapter device for interfacing a medical camera with a processing device. In addition to signal conversion, the signal adapter device is configured to ensure a mechanically secure connection to the processing device, thereby reducing the risk of accidental disconnection. The signal adapter device comprises:
a first interface, which is configured to connect to a medical camera via a first connector and to receive video signals in at least one first signaling format;
a second interface, which is configured to connect to a processing device via a second connector and to output converted video signals in a second signaling format, wherein the first signaling format is different from the second signaling format;
a processing unit, which is electrically connected to both interfaces and configured to:
   receive video signals from the medical camera via the first interface;
   transmit converted video signals to the processing device via the second interface;
   process and convert control signals bidirectionally between the first and second interfaces; and
   a housing, which encloses the processing unit and at least a portion of the first and second interfaces. The housing further comprises a locking mechanism associated with the second interface, which is configured to mechanically secure the connection between the signal adapter device and the processing device to prevent unintended disconnection.

In some implementations, the locking mechanism comprises a release mechanism, which may include at least one of a release button, a lever, or a slider to disengage the second interface from the processing device.

The first interface may comprise a socket configured to receive a plug of a cable connected to the medical camera, wherein the socket is positioned within an interior space of the housing. Additionally, the second interface may comprise a protruding plug, which is configured to be inserted into a corresponding socket of the processing device.

In certain implementations, the locking mechanism is integrated into the protruding plug of the second interface, thereby securing the connection to the processing device.

The first interface may also be configured to release a connected plug when a pulling force exceeding a predefined threshold is applied to the plug.

The housing may further comprise a widened structure extending along the direction of the second interface's contacts. This widened structure is arranged in such a way that it reduces lateral movement of the signal adapter device when the first interface is connected or disconnected. In some implementations, the widened structure is in indirect mechanical contact with the housing of the processing device, thereby contributing to overall stability.

According to a third aspect, the present invention provides a signal adapter device for interfacing a medical camera with a processing device. In addition to signal conversion and mechanical stability, the signal adapter device incorporates heat dissipation structures to manage the thermal energy generated by the processing unit during operation. The signal adapter device comprises:
a first interface, which is configured to connect to a medical camera via a first connector;
a second interface, which is configured to connect to a processing device via a second connector;
a processing unit, which is disposed within the signal adapter device and configured to convert video signals received via the first interface into signals suitable for transmission via the second interface; and
a housing, which encloses the processing unit and at least a portion of the first and second interfaces.

The housing further comprises heat dissipation structures, which are configured to dissipate heat generated by the processing unit.

In some implementations, the heat dissipation structures are configured to dissipate thermal energy collected from at least one of the first interface and the second interface.

The heat dissipation structures may be thermally connected to the first interface via a heat-conducting element, which establishes a thermal path from a camera connected to the first interface.

The housing may comprise an extended dimension in the direction of the second interface's contacts, which is arranged in such a way that it contributes to mechanical stability and facilitates heat dissipation by providing an increased thermal exchange surface.

In some implementations, the housing includes internal heat dissipation elements in thermal contact with the processing unit.

Additionally, the heat dissipation structures may comprise heat sinks (passive cooling elements) positioned along the extended dimension of the housing. Due to their increased surface area, these heat sinks contribute to passive cooling by promoting heat dissipation.

The present invention thus provides a signal adapter device that enables compatibility between legacy medical cameras and modern processing devices. By incorporating a processing unit for signal conversion, a mechanical locking mechanism, and heat dissipation structures, the signal adapter device ensures reliable signal transmission, secure physical connection, and effective thermal management.

The combination of these features allows medical professionals to integrate existing imaging devices into newer systems without compromising functionality. The described implementations may be applied individually or in combination, depending on specific application requirements.

The preceding is a summary of the disclosure to provide an understanding of some aspects of the disclosure. As will be appreciated, other aspects, embodiments, and configurations of the disclosure are possible utilizing, alone or in combination, one or more of the features set forth above or described in detail below.

Numerous additional features and advantages are described herein and will be apparent to those skilled in the art upon consideration of the following Detailed Description and in view of the accompanying figures.

### BRIEF DESCRIPTION OF THE DRAWINGS

The accompanying drawings illustrate embodiments of the present invention and are provided for illustrative purposes only. They should not be interpreted as limiting the invention to the precise configurations shown, as modifications and variations may be implemented within the scope of the invention. Further features and advantages will become apparent from the following, more detailed, description of the various aspects, embodiments, and configurations of the disclosure, as illustrated by the drawings referenced below.
FIG. 1 is a schematic representation of a signal adapter device, showing its connection to a medical camera via a first interface and to a processing device via a second interface.
FIG. 2 is a perspective view of the signal adapter device, illustrating its housing, second interface, and mechanical locking mechanism.
FIG. 3 is an exploded view of the signal adapter device, showing its internal components, including the processing unit, circuit assembly, and locking mechanism.

The drawings serve to provide a better understanding of the invention, but various modifications in terms of mechanical structure, electronic configuration, and cooling mechanisms are possible without departing from the scope of the invention.

### DETAILED DESCRIPTION

The following detailed description provides various embodiments of the present invention. The embodiments described herein are presented as illustrative examples and are not intended to be limiting. Various modifications and alternative implementations may be made without departing from the scope of the invention, as defined by the claims. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Reference will now be made to the accompanying drawings, where applicable, to provide a more comprehensive understanding of the invention. Like reference numerals in different figures indicate like elements, unless otherwise specified.

In general, the signal adapter device described herein enables compatibility between medical cameras using legacy signaling formats and modern processing devices. The signal adapter device provides signal conversion, mechanical securing, and thermal management to ensure reliable operation in clinical environments. Various embodiments may incorporate one or more of these features, depending on the specific application requirements.

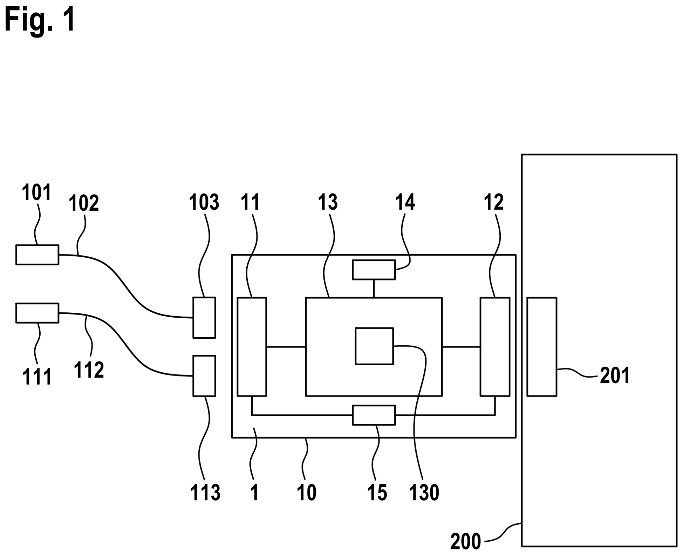

FIG. 1 illustrates an exemplary embodiment of a signal adapter device 1 configured to interface with medical cameras and a processing device. The signal adapter device 1 includes a first interface 11, which is designed to receive video signals from an external medical camera.

In the illustrated embodiment, the first interface 11 is configured to connect to at least one of a first medical camera 101 or a second medical camera 111. The first medical camera 101 is connected via a cable 102 that terminates in a plug connector 103, which is inserted into the first interface 11 of the signal adapter device 1. Alternatively, the second medical camera 111 may be connected via a cable 112 with a corresponding plug connector 113 that is also designed to interface with the first interface 11.

While FIG. 1 illustrates two medical cameras 101 and 111 as examples, it should be understood that the system is not limited to these specific configurations. The first interface 11 may be compatible with any suitable medical camera that utilizes a plug connector 103 or 113 matching the first interface 11 of the signal adapter device 1.

In some implementations, the first medical camera 101 and/or the second medical camera 111 may be commercially available medical cameras, such as those manufactured by Karl Storz. Specific models include the HD Cystoscope (e.g., models 11272VH, 11272VH-TL, 11272VHU, 11272VHU-TL), the Flex-XC (e.g., models 11278VS, 11278VSU, 11278VSE, 11278VSUE), and the IMAGE1 S HX series (e.g., models TH110, TH111).

Additionally, the connection between the first interface 11 and the corresponding plug connector 103 or 113 of the medical camera 101 or 111 may, in certain embodiments, utilize an X-LINK connection (Part Number: TC301), as employed in specific medical camera systems by KARL STORZ.

The connection between the first interface 11 and the corresponding plug connector 103 or 113 of the medical camera 101 or 111 is configured to transmit video signals from the connected camera 101, 111 to the signal adapter device 1. In some implementations, this connection may also support the (optionally bidirectional) transmission of control signals, allowing to send commands to the medical camera 101 or 111. These control signals may be used to adjust camera parameters such as exposure, focus, white balance, Illumination Light Control, Reprogramming of Camera Memory Chips, etc.

Additionally, the connection between the first interface 11 and the medical camera 101 or 111 may also be configured to provide electrical power to the connected camera.

In addition to the first interface 11, the signal adapter device 1 further comprises a second interface 12, which is configured to establish a connection to a processing device 200. This connection is designed as a pluggable interface, preferably comprising a plug connector on the signal adapter device 1 and a corresponding connector 201 on the processing device 200.

In some implementations, this connection may be based on a specialized connector with a specific internal configuration, designed for compatibility with certain medical imaging systems.

Through the second interface 12, video signals are transmitted or exchanged between the signal adapter device 1 and the processing device 200. The video signals transmitted through this connection may be different from those transmitted between the medical camera 101 or 111 and the first interface 11.

Additionally, the second interface 12 may support the exchange of control signals between the signal adapter device 1 and the processing device 200. The format of these control signals may also differ from the format used for communication between the medical camera 101 or 111 and the first interface 11.

Furthermore, the second interface 12 may be configured to receive electrical power from the processing device 200. In some implementations, the signal adapter device 1 may further process the received power, for example, by adjusting its voltage or current characteristics before supplying it to a connected medical camera via the first interface 11.

The signal adapter device 1 comprises a processing unit 13 disposed within its housing 10. The processing unit 13 is configured to convert video signals received via the first interface 11 into signals suitable for transmission via the second interface 12.

In some implementations, the processing unit 13 comprises a field-programmable gate array (FPGA) 130, which serves as the core processing element for handling signal conversion. The specific type of FPGA 130 may vary depending on the performance and compatibility requirements of the signal adapter device 1. One suitable FPGA model is the Artix-7 series from Xilinx, which provides a balance of processing power and efficiency for signal conversion tasks. Alternatively, the processing unit 13 may include another type of signal processing component with similar or equivalent functionality , such as an application-specific integrated circuit (ASIC) or a dedicated signal processing unit.

The programming of the processing unit 13 or the FPGA 130 may be configured in different ways. In some implementations, the programming is set during manufacturing and remains fixed and unchangeable. Alternatively, the programming of the FPGA 130 may be modifiable, for example, by adding, replacing, or storing a program code in a memory component within the processing unit 13 or the FPGA 130.

In embodiments where reprogramming is possible, the processing unit 13 may be configured to receive programming or firmware updates through the second interface 12, directly from the processing device 200. For instance, the processing device 200 may switch the signal adapter device 1 into a programming mode and subsequently transmit the corresponding program code.

Alternatively, the signal adapter device 1 may comprise a dedicated programming interface 14, which allows the processing unit 13 to be programmed independently of the second interface 12.

The first interface 11 of the signal adapter device 1 is configured to receive video signals from a connected medical camera 101 or 111. In some implementations, the cameras output video signals are in a Digital Visual Interface (DVI) format or a Low-Voltage Differential Signaling (LVDS) format, which are standardized formats commonly used in medical imaging systems. However, in certain cases, proprietary camera systems may utilize modified or adapted versions of these formats, which may include variations in encoding, timing, or signal modulation.

The second interface 12, which connects the signal adapter device 1 to the processing device 200, is configured to transmit the converted video signals in a format compatible with the processing device. In many cases, the processing device 200 may require video signals in a Multi-Gigabit Transceiver (MGT) format or a format similar to MGT, which differs from the formats used by the cameras.

The electrical contacts at the first interface 11 and the second interface 12 may be designed to accommodate the respective signaling formats, ensuring that video signals can be properly received, transmitted, and processed.

In addition to video signals, the first interface 11 and the second interface 12 may also support the exchange of control signals between the connected devices. In some implementations, the control signals may be embedded within the respective video signal format using existing signaling protocols. Alternatively, the control signals may be transmitted using separate protocols or dedicated signal formats. Depending on the system configuration, control signals may be transmitted over the same signal lines as the video signals or via separate dedicated signal paths within the interfaces.

In some implementations, the conversion of video and/or control signals by the processing unit 13 may include multiplexing or demultiplexing techniques, allowing multiple data streams to be transmitted over a shared communication channel and later separated for processing.

The processing unit 13, which is disposed within the signal adapter device 1, is responsible for converting the video and control signals exchanged between the first interface 11 and the second interface 12. This conversion ensures that video signals provided by a connected medical camera 101 or 111 can be processed by the processing device 200, even if the respective signal formats differ.

In some implementations, the processing unit 13 includes an FPGA 130 or another programmable signal processing component, which is configured to handle real-time conversion of video data. Depending on the formats involved, this conversion may include:
- reformatting the video signal structure, such as adjusting pixel encoding, frame timing, or synchronization parameters;
- signal modulation or demodulation, for example, adapting the voltage levels, clocking schemes, or encoding methods; and/or
- protocol adaptation, ensuring that metadata, synchronization signals, or embedded control data are correctly interpreted and transferred.

The processing unit 13 is also capable of handling control signal conversion, ensuring that command signals exchanged between the camera 101 or 111 and the processing device 200 remain compatible. In some implementations, control signals received from the processing device 200 may be reformatted before transmission to the camera, while control signals originating from the camera may be adjusted to align with the expected format of the processing device 200.

Furthermore, in systems where multiplexing techniques are employed, the processing unit 13 can extract embedded control signals from a video data stream or integrate control signals into the video transmission path. This allows for efficient use of available communication channels while maintaining a structured signal flow.

The signal processing functions performed by the processing unit 13 may be implemented in hardware, firmware, or software. If the processing unit 13 includes a programmable logic component, such as an FPGA 130, it may allow for updates and modifications to support additional signal formats or system adaptations.

The first interface 11 of the signal adapter device 1 is designed to support multiple different video signal formats from various medical cameras. Regardless of the specific format received at the first interface 11, the processing unit 13 is configured to convert the signal into the required format for transmission via the second interface 12.

To enable this, the processing unit 13 includes software-based conversion algorithms, which allow it to handle a wide range of video and control signal formats. The software governing these conversions may be stored within the processing unit 13 and, in some implementations, may be updated at a later stage to support additional formats as needed. The update may be performed via the second interface 12 from the processing device 200 or via a dedicated programming interface 14.

In some implementations, the processing unit 13 is configured to automatically detect the signal format received at the first interface 11. This detection allows the processing unit 13 to dynamically adjust the conversion settings to ensure compatibility with the expected format at the second interface 12.

The automatic detection of the signal format may be performed in various ways, including but not limited to:
- analyzing the electrical characteristics of the incoming signal, such as voltage levels, clocking, or synchronization patterns;
- evaluating a dedicated signaling mechanism, where a specific signal or voltage level at one or more pins of the first interface 11 indicates the format of the transmitted video signals; and/or
- detecting a mechanical encoding feature on the connector, where the plug 103 or 113 of the medical camera 101 or 111 includes a physical element that provides an identifier for the connected device.

If a mechanical encoding system is used, the signal adapter device 1 may comprise a dedicated detection unit, which evaluates the mechanical encoding and provides the corresponding information to the processing unit 13. This detection unit may be implemented in various forms, such as a simple switch that toggles between two predefined positions based on the connector type, or a more complex identification mechanism, capable of distinguishing multiple different connector variants and signaling the detected format to the processing unit 13.

Additionally, the adaptive conversion capabilities of the processing unit 13 may also include the transformation of control signals in both directions, ensuring that the correct protocol is used for communication between the medical camera 101 or 111 and the processing device 200. In some implementations, the processing unit 13 may adjust the control signal format based on the detected camera type, ensuring that the appropriate signaling method is used for command transmission and reception. This adaptation may be performed dynamically in response to the detected electrical characteristics, dedicated signaling mechanisms, or mechanical encoding features of the connector.

The second interface 12 of the signal adapter device 1 is generally designed to conform to a known standard format for connecting to the processing device 200. This ensures compatibility with widely used medical imaging systems. However, to provide greater flexibility, the signal adapter device 1 may also be configured to support different processing devices 200, which may operate with varying configurations or software versions.

To accommodate such variations, the processing unit 13 may be configured to adapt dynamically to the specific requirements of the connected processing device 200. For example, upon insertion of the signal adapter device 1 into the second interface 12, an initial data exchange may take place between the signal adapter device 1 and the processing device 200. This communication can serve to:
- identify the expected video signal format for transmission from the signal adapter device 1 to the processing device 200;
- determine the appropriate format for control signal exchange in both directions; and/or
- configure interface settings based on the detected capabilities and requirements of the processing device 200.

The exchange of configuration data may occur using a dedicated communication protocol that allows the processing unit 13 to negotiate compatible settings with the processing device 200. In some implementations, this initial handshake may be based on standardized protocols or on proprietary communication methods specific to the processing device 200.

By implementing such an adaptive configuration mechanism, the signal adapter device 1 can ensure seamless integration with different processing devices 200, even if they operate with different firmware versions, software configurations, or hardware requirements.

The medical cameras 101 or 111 connected to the first interface 11 of the signal adapter device 1 typically receive their power supply from the processing device 200. In the simplest implementation, the power supplied by the processing device 200 may be directly passed through the signal adapter device 1 to the connected camera. In such cases, the processing device 200 may already include an appropriate voltage conversion unit and, if necessary, an isolation mechanism, such as galvanic isolation, to ensure safe power delivery to the camera.

Alternatively, the signal adapter device 1 may be configured to adjust the power supply to meet the requirements of the connected medical camera 101 or 111. In such implementations, the signal adapter device 1 includes a voltage converter 15, which converts the power received from the processing device 200 into a voltage level suitable for the connected camera. The voltage conversion performed by the voltage converter 15 may be predefined in a fixed configuration or dynamically adjusted based on the detected power requirements of the connected medical camera. In the latter case, the signal adapter device 1 can determine the necessary voltage level by analyzing the electrical characteristics of the connected camera, evaluating dedicated signaling mechanisms that indicate the required voltage, or detecting mechanical encoding features on the camera connector that provide an identifier for the appropriate power level.

In some implementations, the signal adapter device 1 may also include one or more isolation mechanisms to enhance electrical safety, particularly in medical applications. The power supply can be galvanically isolated through transformers, isolating converters, or similar components, preventing direct electrical coupling between the processing device 200 and the connected medical camera. Additionally, signal transmission for video and/or control data can be isolated using optocouplers or similar isolation devices within the processing unit 13. By implementing these isolation techniques, the signal adapter device 1 can help protect both the medical camera and the processing device 200 from electrical faults, leakage currents, and potential interference, ensuring stable and reliable operation in sensitive environments.

In summary, the signal adapter device 1 ensures proper power supply and electrical isolation while facilitating signal conversion between different video and control signal formats. Depending on the implementation, the signal adapter device 1 may either pass through the power supply from the processing device 200 to the connected medical camera 101 or 111, or it may actively regulate the voltage using an integrated voltage converter 15. In cases where an adaptive power configuration is required, the signal adapter device 1 can determine the appropriate voltage level based on electrical characteristics, signaling mechanisms, or mechanical encoding features of the connected camera.

To enhance electrical safety, particularly in medical environments, the signal adapter device 1 may incorporate galvanic isolation for both power supply and data transmission, which can be implemented using transformers, isolating converters, or optocouplers. These measures contribute to reliable operation by protecting the medical camera 101, 111 and the processing device 200 from electrical faults, leakage currents, or interference.

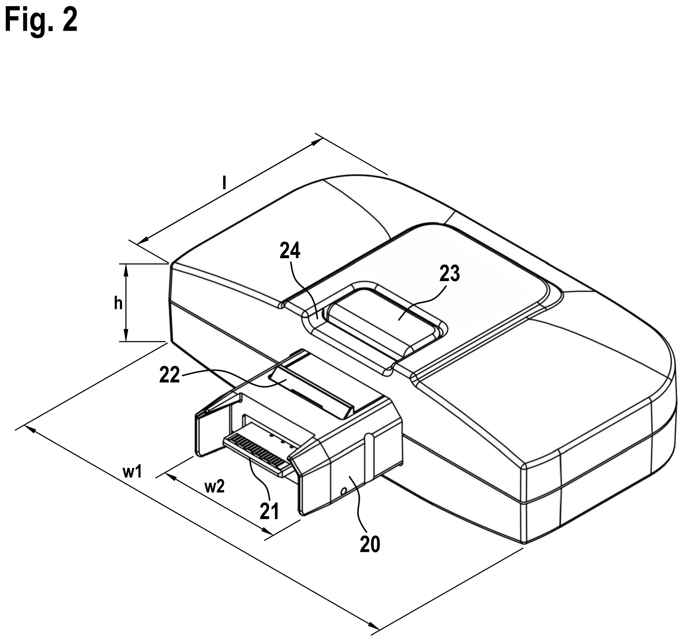

FIG. 2 illustrates a perspective external view of the signal adapter device 1, showing the housing 10 and the elements related to the mechanical connection between the signal adapter device 1 and the processing device 200. The signal adapter device 1 comprises a protruding connector section 20, which forms part of the second interface 12 and extends outward from the housing.

The connector section 20 includes electrical contacts 21, which are arranged to establish an electrical connection with corresponding contacts of the connector 201 on the processing device 200. The shape and dimensions of the connector section 20 are configured so that it can be inserted into a corresponding recess or slot in the processing device 200, ensuring a precise alignment during connection.

By allowing the connector section 20 to extend into the corresponding recess, the connection between the signal adapter device 1 and the processing device 200 is mechanically stabilized, reducing the likelihood of unintended lateral movement or misalignment during use.

To ensure a secure connection between the signal adapter device 1 and the processing device 200, a locking mechanism is provided. This locking mechanism, which is particularly implemented as a mechanical locking feature, prevents the signal adapter device 1 from being unintentionally detached from the processing device 200.

Such a locking mechanism offers several advantages. First, it ensures that the signal adapter device 1 remains securely attached to the housing of the processing device 200 over extended periods, reducing the risk of the signal adapter device 1 being misplaced when not in use. Additionally, the locking mechanism provides a practical benefit during operation: If the plug 103 or 113 of a medical camera 101 or 111 is removed from the first interface 11, the signal adapter device 1 remains securely connected to the processing device 200. This stabilizes the signal adapter device 1 and facilitates the disconnection of the camera plug 103 or 113, as the signal adapter device 1 does not shift or move when the camera 101, 111 is disconnected.

Unlike the mechanical locking mechanism securing the signal adapter device 1 to the processing device 200, no such locking feature is provided at the first interface 11, where the medical camera 101 or 111 is connected via the plug 103 or 113. This design allows the plug 103 or 113 to be disconnected more easily in response to external forces, preventing damage to the connected devices or the cabling.

In some implementations, the connection between the first interface 11 and the plugs 103 or 113 of the camera cables 102 or 112 is specifically designed to release when a predefined pulling force is applied. This ensures that, in the event of an unintended force exerted on the cable 102 or 112, the plug 103 or 113 is safely disconnected from the first interface 11, while the signal adapter device 1 remains securely attached to the processing device 200.

This controlled release mechanism helps prevent mechanical stress on the signal adapter device 1 and the processing device 200 and reduces the risk of damage to the electrical contacts of the interfaces. Additionally, it enhances operational safety by avoiding abrupt movements of the processing device 200 in case of an accidental pull on the camera cable.

Various types of mechanical locking mechanisms may be used to secure the signal adapter device 1 to the processing device 200. One exemplary implementation is illustrated in FIG. 2, where a locking element 22 extends from at least one side of the protruding connector section 20, which forms part of the second interface 12. The locking element 22 may be designed as a gripper, latch, pin, or similar feature, and is biased outward by a spring force or a similar mechanism.

When the signal adapter device 1 is inserted into the corresponding recess in the connector 201 of the processing device 200, the locking element 22 automatically engages with a corresponding depression or groove within the connector 201. This engagement prevents unintended disconnection of the signal adapter device 1 and ensures a stable connection between the signal adapter device 1 and the processing device 200.

To release the locking mechanism, an additional mechanical component may be provided. In the illustrated implementation, a release button 23 is integrated into the signal adapter device 1. When the release button 23 is pressed, it moves the locking element 22 inward (i.e., the locking element 22 is retracted), thereby disengaging it (or them in the case of multiple locking elements) from the corresponding recess in the connector 201. This allows the signal adapter device 1 to be safely detached from the processing device 200 without requiring excessive force.

For accessibility, the release button 23 may be positioned at an opening 24 in the housing 10 of the signal adapter device 1, allowing easy manual operation.

FIG. 2 further illustrates the geometrical dimensions of the signal adapter device 1. The length of the signal adapter device 1 is denoted as 1, the height as h, and the width of the housing 10 as w1. The width of the protruding connector section 20, which extends from the housing 10 and forms part of the second interface 12, is denoted as w2.

In one possible implementation, the height h of the housing 10 may be freely chosen depending on design requirements. However, in preferred configurations, the height h is not significantly greater than the height of the connector section 20, ensuring a compact form factor. For example, the height h may be between 100% and 150% of the height of the connector section 20.

Regarding the width dimension, different design variations are possible. In some implementations, the width w1 of the signal adapter device 1 housing 10 is substantially larger than the width w2 of the connector section 20. For instance, in one exemplary configuration, the width w1 may be at least three times the width w2, although this is not a strict limitation, and other width ratios, particularly larger widths, are also possible.

By designing the signal adapter device 1 with an increased width w1, lateral movement or tilting of the signal adapter device 1 within the connector 201 of the processing device 200 can be minimized or significantly reduced. This enhances the overall mechanical stability of the signal adapter device 1 when connected to the processing device 200, ensuring a secure and stable attachment during operation.

In some implementations, the stabilization of the signal adapter device 1 may be further improved by a direct mechanical contact between the housing 10 of the signal adapter device 1 and the housing of the processing device 200. This direct contact can provide additional support, reducing mechanical stress on the connector section 20 and further preventing unintended movement of the signal adapter device 1 once inserted into the connector 201.

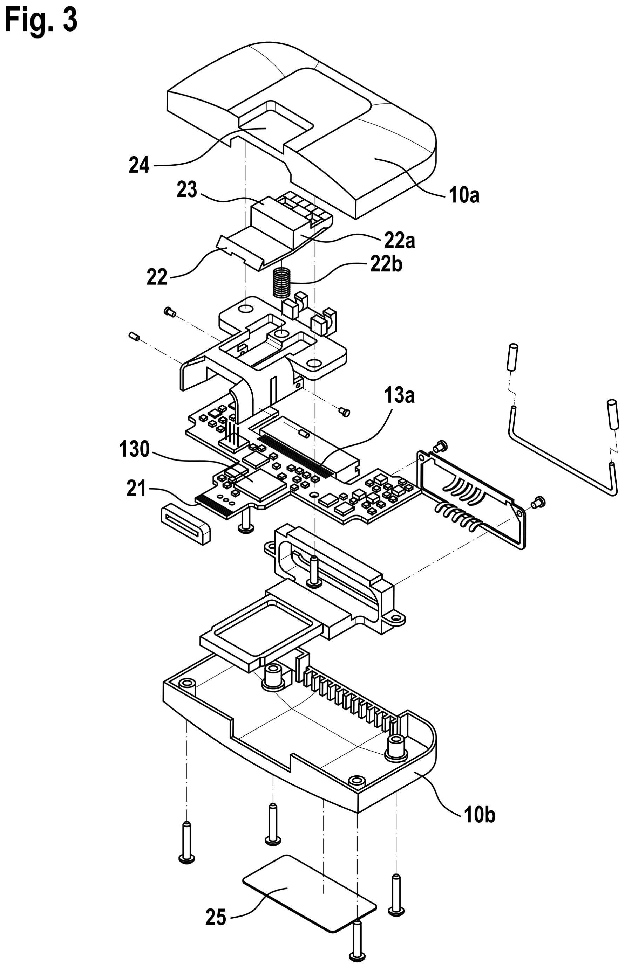

FIG. 3 illustrates an exploded view of the signal adapter device 1, showing its internal components and assembly structure. The housing 10 of the signal adapter device 1 is composed of two half-shells 10a and 10b, which enclose the internal components. These half-shells may be made of or include plastic material, depending on the specific application. In some implementations, the housing 10 may be partially or fully metallized to provide electromagnetic shielding, thereby improving electromagnetic compatibility (EMC). Additionally, metallic components may also serve as heat dissipation structures, enhancing thermal management within the signal adapter device 1.

The two half-shells 10a and 10b can be joined in various ways, including gluing, snapping, or screwing. The embodiment shown in FIG. 3 illustrates a screw connection, ensuring a secure and serviceable assembly of the signal adapter device 1.

Inside the signal adapter device 1 housing 10, a circuit assembly 13a is arranged, which includes the processing unit 13, in particular an FPGA 130, for handling the conversion of video and control signals between the first interface 11 and the second interface 12.

FIG. 3 also illustrates an exemplary implementation of the locking mechanism 22, which is designed to secure the signal adapter device 1 to the processing device 200. The locking mechanism 22 may be realized using a component 22a, which comprises both the locking pin or latch that engages with a corresponding recess in the housing of the processing device 200, as well as the release button 23 for disengagement. As further shown in FIG. 3, the locking mechanism 22 is biased into its engaged position by a spring 22b, ensuring that the locking element remains in place unless actively released (or: retracted) by pressing the button 23.

Depending on the application, alternative implementations of the locking mechanism 22 are possible. Different types of springs, locking structures, or release mechanisms may be used to achieve the desired locking and unlocking functionality while maintaining ease of use and mechanical stability.

FIG. 3 also illustrates components that may contribute to an improved thermal management by dissipating the heat generated inside the signal adapter device 1. During operation, various internal components, including the processing unit 13 and the circuit assembly 13a, produce heat, which should be effectively dissipated to maintain stable performance and prevent overheating.

To achieve this, the housing 10, particularly the upper shell 10a and/or lower shell 10b, may be designed to function as a heat dissipation structure. These components may be made of or include thermally conductive materials, such as metal plates or other metallic elements, allowing heat to be transferred away from the internal components to the external surface of the signal adapter device 1.

In some implementations, more complex cooling structures may be integrated into the housing 10. For instance, cooling fins or other passive cooling elements may be incorporated into the upper shell 10a and/or the lower shell 10b to increase the effective heat dissipation surface area. These passive cooling elements can enhance heat exchange with the surrounding environment, improving overall thermal performance.

Additionally, thermal dissipation may be further supported by the direct mechanical contact between the housing 10 of the signal adapter device 1 and the housing of the processing device 200. This contact provides an additional thermal pathway, allowing heat to be transferred from the signal adapter device 1 to the processing device 200. In some implementations, the processing device 200 may already incorporate a dedicated cooling system, such as internal heat sinks or active cooling elements. By leveraging this thermal connection, the signal adapter device 1 can utilize the cooling capacity of the processing device 200, further improving heat dissipation.

For applications where higher amounts of heat must be dissipated, the signal adapter device 1 may also include an active cooling system, such as one or more small fans, to provide additional airflow and accelerate heat removal.

By implementing such cooling strategies, the signal adapter device 1 ensures stable operation and prolonged durability, even when handling high-performance signal processing tasks.

In certain applications, the medical camera 101 or 111, when connected to the signal adapter device 1, may generate significant heat due to internal electronics and built-in light sources. Elevated temperatures in the camera are particularly undesirable in medical examinations, where patient safety is very important. To address this, the signal adapter device 1 may include a dedicated component located in the vicinity of the first interface 11. This component is designed to capture and conduct thermal energy from the camera 101 or 111, which is transmitted along the cable 102 or 112 to the plug connector 103 or 113.

The captured heat is then transferred to an integrated cooling structure 25 within the signal adapter device 1. The cooling structure 25 may comprise passive elements such as heat sinks, cooling fins, or other thermally conductive components, or even active cooling elements, to effectively dissipate the thermal energy. In this way, the signal adapter device 1 not only performs signal conversion but also assists in maintaining the camera at a safe operating temperature during medical procedures.

Summarizing, the present invention relates to a signal adapter device designed to interface a medical camera with a processing device, ensuring compatibility between different signal formats. The signal adapter device 1 performs signal conversion, mechanical stabilization, and thermal management, allowing seamless integration of legacy camera systems with modern processing units.

The signal adapter device 1 comprises a first interface that is configured to receive video signals from a medical camera in one of several possible formats, such as DVI or LVDS, or proprietary variations thereof. A second interface establishes a connection to the processing device, which may expect signals in a different format, such as MGT-based signaling. The processing unit, which may include an FPGA or a functionally equivalent processing component, converts the video and control signals bidirectionally between the first and second interfaces.

Additionally, the signal adapter device can automatically detect the format of the input signal and adjust the conversion settings accordingly. This detection may be based on electrical characteristics, dedicated signaling mechanisms, or mechanical encoding features at the first interface. The processing unit may be programmable, either during manufacturing or via firmware updates through the second interface or a dedicated programming interface.

The signal adapter device is housed in a protective casing, which may be composed of two half-shells made of plastic, metal, or a combination thereof. The second interface features a protruding connector section that engages with a corresponding slot on the processing device, ensuring precise alignment and secure connection.

To prevent unintended disconnection, the signal adapter device incorporates a mechanical locking mechanism, which may include a spring-loaded locking element that engages with a corresponding recess in the processing device's connector. A release button allows for controlled disengagement, facilitating easy removal of the signal adapter device 1 when required.

Conversely, at the first interface, no mechanical locking is provided, allowing the camera plug to disconnect safely if excessive force is applied to the camera cable. This feature prevents mechanical stress on the signal adapter device and connected components while enhancing safety in clinical environments.

The signal adapter device also incorporates thermal management features to dissipate heat generated during operation. The housing may function as a heat dissipation element, incorporating thermally conductive materials or dedicated cooling structures, such as heat sinks or cooling fins.

Additionally, heat from the camera itself may be transferred via the camera cable to the signal adapter device, where a heat-absorbing element near the first interface captures the thermal energy and redirects it to cooling structures within the signal adapter device. In some implementations, the signal adapter device may be in thermal contact with the housing of the processing device, enabling additional heat dissipation through the processing unit's cooling system.

For higher thermal loads, the signal adapter device may incorporate an active cooling system, such as small fans, to enhance heat dissipation.

The signal adapter device can pass through power from the processing device to the camera or regulate the voltage using an integrated voltage converter. In some implementations, the signal adapter device automatically adjusts the power supply based on the detected camera requirements.

To ensure electrical safety, galvanic isolation may be provided between the first and second interfaces. This can be implemented using isolation transformers for power transfer or optocouplers for signal transmission, minimizing the risk of electrical faults and interference.

The signal adapter device is designed for mechanical stability, with an extended housing width relative to the connector width, minimizing lateral movement. In some implementations, the housing is in direct mechanical contact with the processing device, further enhancing structural rigidity.

Concluding, the signal adapter device provides a robust and flexible solution for integrating legacy medical cameras with modern processing units. Through advanced signal conversion, secure mechanical fixation, and efficient thermal management, the signal adapter device ensures reliable performance, safety, and ease of use in medical applications.

The foregoing description provides various embodiments of the present invention. However, these embodiments are intended as illustrative examples and should not be construed as limiting the scope of the invention, which is defined by the accompanying claims. Modifications, adaptations, and variations that do not depart from the essence of the invention may be implemented within the claimed scope.

It should be understood that the specific implementations described herein, including the mechanical locking mechanism, signal conversion process, power regulation, and thermal management features, may be modified or substituted based on application-specific requirements. For example, different locking mechanisms, alternative electronic components, or various cooling structures may be employed while still achieving the intended functionality of the signal adapter device.

The terminology used herein is for descriptive purposes only and should not be interpreted as limiting. For instance, references to "processing unit" encompass any suitable signal processing hardware, including but not limited to FPGAs, ASICs, or other programmable logic devices. Similarly, references to "video signal formats" include standardized and proprietary variations.

Furthermore, individual features described in relation to a specific embodiment may, where applicable, be combined with features from other embodiments, unless explicitly stated otherwise. The order of steps, structural configurations, and signal processing techniques may be altered or interchanged as necessary to optimize performance for different
implementations.

As used herein, the singular forms "a," "an," and "the" are intended to include the plural forms as well, unless the context clearly indicates otherwise. It will be further understood that the terms "include," "including," "includes," "comprise," "comprises," and/or "comprising," when used in this specification, specify the presence of stated features, integers, steps, operations, elements, and/or components, but do not preclude the presence or addition of one or more other features, integers, steps, operations, elements, components, and/or groups thereof. The term "and/or" includes any and all combinations of one or more of the associated listed items. The use of "including," "comprising," or "having" and variations thereof herein is meant to encompass the items listed thereafter and equivalents thereof as well as additional items. Accordingly, the terms "including," "comprising," or "having" and variations thereof can be used interchangeably herein.

Accordingly, the present invention should be considered in its broadest reasonable interpretation, consistent with the disclosure and as defined by the scope of the claims.

## Claims

1. Signal adapter device (1) for interfacing a medical camera (101; 111) with a processing device (200), the signal adapter device (1) comprising:
a first interface (11) configured to connect to a medical camera (101; 111) via a first connector (103; 113), the medical camera (101; 111) providing signals in at least one first signaling format;
a second interface (12) configured to connect to a processing device (200) via a second connector (201), the processing device (200) requiring signals in a second signaling format, wherein the first signaling format is different from the second signaling format;
a processing unit (13) disposed within the signal adapter device (1) and electrically connected to:
the first interface (11), to receive video signals in the at least one first signaling format and to transmit control signals to the camera (101; 111); and
the second interface (12), to transmit converted video signals in the second signaling format and to receive control signals from the processing device (200);
wherein the processing unit (13) is configured to convert the video signals received via the first interface (11) into signals in the second signaling format and to convert control signals received via the second interface (12) into signals suitable for the first interface (11); and
a housing (10) enclosing the processing unit (13) and at least a portion of the first and second interfaces (11; 12).

2. Signal adapter device (1) according to claim 1, wherein the first signaling format is based on at least one of a Digital Visual Interface (DVI) signal and a Low-Voltage Differential Signaling (LVDS) signal, and/or the second signaling format is based on a Multi-Gigabit Transceiver (MGT) standard.

3. Signal adapter device (1) according to claim 1 or 2, wherein the processing unit (13) comprises a field-programmable gate array (FPGA) (130) or another programmable signal converter configured to perform the signal conversion between the first interface (11) and the second interface (12), wherein preferably the FPGA (130) or programmable converter is configured to receive programming or firmware updates via at least one of a dedicated programming interface or the second interface (12).

4. Signal adapter device (1) according to one of the previous claims, wherein the processing unit (13) is configured to detect the signal format received via the first interface (11) and automatically adjust the conversion settings.

5. Signal adapter device (1) according to one of the previous claims, wherein the first interface (11) provides power to the camera (101; 111) via the first connector (103; 113), and the signal adapter device (1) includes a voltage converter (15) configured to adjust the voltage received from the second interface (12) to a level suitable for operating the camera (101; 111).

6. Signal adapter device (1) according to one of the previous claims, further comprising an isolation circuit configured to provide galvanic isolation between the first interface (11) and the second interface (12).

7. Signal adapter device (1) for interfacing a medical camera (101; 111) with a processing device (200), the signal adapter device (1) comprising:
a first interface (11) configured to connect to a medical camera (101; 111) via a first connector (103; 113), the medical camera (101; 111) providing signals in at least one first signaling format;
a second interface (12) configured to connect to a processing device (200) via a second connector (201), the processing device (200) requiring signals in a second signaling format, wherein the first signaling format is different from the second signaling format;
a processing unit (13) disposed within the signal adapter device (1) and electrically connected to:
the first interface (11), to receive video signals in the at least one first signaling format and to transmit control signals to the camera (101; 111); and
the second interface (12), to transmit converted video signals in the second signaling format and to receive control signals from the processing device (200); and
a housing (10) enclosing the processing unit (13) and at least a portion of the first and second interfaces (11; 12), the housing (10) comprising a locking mechanism (22) associated with the second interface (12), the locking mechanism (22) being configured to mechanically secure the connection between the signal adapter device (1) and the processing device (200) to prevent unintended disconnection.

8. Signal adapter device (1) according to claim 7, wherein the locking mechanism (22) comprises a release mechanism, including at least one of a release button (23), a lever, or a slider, to disengage the second interface (12) from the processing device (200).

9. Signal adapter device (1) according to claim 7 or 8, wherein the first interface (11) comprises a socket configured to receive a plug of a cable (102; 112) connected to the medical camera (101; 111), the socket being positioned within an interior space of the housing (10); and/or
wherein the second interface (12) comprises a protruding plug configured to be inserted into a corresponding socket of the processing device (200), wherein preferably the locking mechanism (22) is integrated into the protruding plug of the second interface (12) to secure the connection to the processing device (200).

10. Signal adapter device (1) according to one of the claims 7 to 9, wherein the first interface (11) is configured to release a connected plug when a pulling force exceeding a predefined threshold is applied to the plug.

11. Signal adapter device (1) according to one of the claims 7 to 10, wherein the housing (10) further comprises a widened structure extending along the direction of the second interface's contacts, wherein the widened structure being configured to minimize lateral movement of the signal adapter device (1) when the first interface (11) is connected or disconnected, wherein preferably the widened structure is configured to form an indirect mechanical contact with the housing of the processing device to enhance stability.

12. Signal adapter device (1) for interfacing a medical camera (101; 111) with a processing device (200), the signal adapter device (1) comprising:
a first interface (11) configured to connect to a medical camera (101; 111) via a first connector (103; 113);
a second interface (12) configured to connect to a processing device (200) via a second connector (201);
a processing unit (13) disposed within the signal adapter device (1) and configured to convert video signals received via the first interface (11) into signals suitable for transmission via the second interface (12); and
a housing (10) enclosing the processing unit (13) and at least a portion of the first and second interfaces (11; 12), the housing (10) comprising heat dissipation structures configured to dissipate heat generated by the processing unit (13).

13. Signal adapter device (1) according to claim 12, wherein the heat dissipation structures are configured to dissipate thermal energy collected from at least one of the first interface (11) and the second interface (12).

14. Signal adapter device (1) according to claim 12 or 13, wherein the heat dissipation structures are thermally connected to the first interface (11) via a heat-conducting element to facilitate heat transfer from a camera (101; 111) connected to the first interface (11).

15. Signal adapter device (1) according to one of the claims 12 to 14, wherein the housing (10) comprises an extended dimension in the direction of the second interface's contacts, configured to improve mechanical stability and enhance thermal dissipation.

16. Signal adapter device (1) according to one of the claims 12 to 15, wherein the housing (10) includes internal heat dissipation elements in thermal contact with the processing unit (13).

17. Signal adapter device (1) according to one of the claims 12 to 16, wherein the heat dissipation structures comprise heat sinks positioned along the extended dimension of the housing (10) to facilitate passive cooling.
